# EUROPEAN PATENT SPECIFICATION

(11) **EP 1 804 745 B1**
(45) Date of publication and mention of the grant of the patent: **01.12.2010**
(21) Application number: 05775874.0
(22) Date of filing: 30.08.2005
(51) Int. Cl.: A61F 5/453

(54) **AN EXTERNAL URINARY CATHETER**
EXTERNER HARNKATHETER
CATHETER URINAIRE EXTERNE

(30) Priority: 30.08.2004 DK 200401306; 16.06.2005 US 690894 P
(43) Date of publication of application: 11.07.2007
(73) Proprietor: Coloplast A/S, 3050 Humlebaek (DK)
(72) Inventor: NIELSEN, Jesper Touborg, DK-2980 Kokkedal (DK)
(74) Representative: Laudrup, Peter
(86) International application number: PCT/EP2005/054252
(87) International publication number: WO 2006/024637

(56) References cited:
- US-A- 3 339 551
- US-A- 3 608 552
- US-A- 3 721 243
- US-A- 3 835 857
- US-A1- 2003 212 375
- US-A1- 2004 034 335

## Description

The present invention relates to an external urinary catheter with a longitudinal axis, said catheter comprising a sheath portion and a tip portion for receiving a connector, said tip portion being attached to one end of said sheath portion and comprising a seizing area intended for seizing and subsequent guidance of said tip portion. The closest prior art is US 2003/212 375 A, which defines the preamble of claim 1.

An external urinary catheter as defined in the introduction is well known in the art and is e.g. described in Applicant's international published applications WO 2004/004796 A1 and WO 91/17728. External urinary catheters are conventionally used in urinary catheter devices for aiding male urinary incontinence and for use in hospitals in connection with treatment and surgery of urethral disorders. Such an external urinary catheter comprises a sheath or body portion enclosing the shaft of the penis, and a tip portion that is provided with a comparatively short discharge tube that via a hose is connected to a urine collection or urinary bag that is a fastened to the bed or the leg of the user. Alternatively, the external urinary catheter, or condom catheter, is arranged externally on the penis and may be a corona urisheath sealing against the foreskin.

The connection of the tip portion and the urinary bag is traditionally obtained by pushing a cone shaped hose connector into a flexible tip connector of the tip portion of the catheter. In order to obtain a safe connection that does not come apart unintentionally, the tip connector and the hose connector have to be pushed together with considerable force. Pulling the connectors apart from each other requires an equally large force.

The hose connector is normally a relatively rigid element that may be easily manipulated by the user- However, the soft, slippery material of the tip portion results in that two strong hands are required in order to connect or release the two parts. Therefore, disabled users most often require help to be able to perform the mounting of the urinary catheter. Such help is costly and since most users consider mounting of urinary catheter to be a private matter, they consider it more or less unpleasant. Furthermore, medical staff or helpers are required to wear gloves when manipulating external urinary catheters. Such gloves are fabricated of a soft and slippery material similar to the material of the urinary catheter and this further aggravates the problem.

As a solution to the described problem several connections based on locking devices have been suggested. However, such devices are bulky, uncomfortable and are not easily manipulated.

Thus, it is the object of the present invention to provide an external urinary catheter of the type mentioned in the introduction, which overcomes or alleviates the described problem.

To meet this object the external urinary catheter is characterized in that said seizing area comprises friction-increasing means according to claim 1.

With such friction-increasing means seizing and manipulating the tip portion is made considerably easier. Also, connecting and releasing of the tip portion and the connector is more readily carried out. Thus, it is possible even for disabled users to handle mounting of the external urinary catheter according to the invention without the need for assistance from a medical staff. As a consequence, users experience a more comfortable mounting and dismounting of the urinary catheter, and medical personnel may be saved.

Moreover, since friction-increasing means may be readily manufactured during the production of the external urinary catheter, the said advantages may be obtained with low or no additional production costs.

Furthermore, such friction-increasing means provides friction in the proper direction when connecting or releasing the tip portion and the hose.

Furthermore, the present invention discloses a method for manufacturing a tip portion for receiving a connector, said tip portion as part of an external urinary catheter comprising a seizing area intended for seizing and subsequent guidance of said tip portion, wherein said seizing area comprises friction-increasing means comprising the step of injection-moulding the tip portion of the catheter into a cavity that is provided with the projections or indentations necessary for producing the friction-increasing means. Injection-moulding advantageously provides a simple, one-step process where the tip portion, usually along with the whole external urinary catheter, is created simultaneously with the friction-increasing means provided thereon. Instead of injection-moulded the tip portion or the whole external urinary catheter can be blow-moulded.

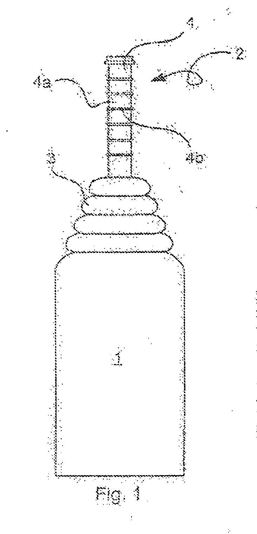

The invention will be explained in detail in the following by means of examples of embodiments with reference to the schematic drawing, in which
Fig. 1 is a first embodiment of the external urinary catheter according to the invention

Fig. 1 shows embodiments of an external urinary catheter according to the invention with a longitudinal axis. Generally, the catheter comprises a circular cylindrical sheath portion 1 for mounting on the penis of a user (not shown) and a trip portion 2 for connecting the urinary catheter to a urinary bag or the like (not shown). The tip portion 2 is attached to the sheath portion 1 at an upper end thereof. In the embodiments shown, the tip portion 2 comprises a flexible bellows portion 3 and a circular cylindrical tip connector 4 for connecting the external urinary catheter to a hose leading to the urinary bag.

The bellows portion 3 comprises four annular parts that may be arbitrarily compressed, whereby the tip connector 4 may be directed at any angle to the longitudinal axis of the external urinary catheter less than approximately 90°. This prevents blockage of the hose leading to the urinary bag and for movement of the catheter independently from the urinary bag.

The tip connector 4 comprises a seising area 4a. for seizing by hand and subsequently guiding the tip portion into connection with a hose connector of the hose leading to the urinary bag. The hose connector may e.g. be in the form of a hollow cone, which is pushed into the circular opening of the tip connector 4. In the embodiments shown, the seising area 4a is the smooth part of the tip connector 4 between the two ends of the tip connector 4. In order to connect the external urinary catheter to the urinary bag, the user seizes the hose connector with one hand and grabs hold of the seising area 4a of the tip connector 4 with the other hand. Then, the user guides the cone of the hose connector into the tip connector 4 and pushes them into a firm grip with each other. In order to facilitate this seizing and guidance operation, the seizing area 4a is disposed on its surface with friction-increasing means 4b.

In the embodiment of Fig. 1 the friction-increasing means 4b are in the form of six ribs extending circumferentially about and perpendicularly to the longitudinal axis of the external urinary catheter. This disposition of the ribs provides for a large frictional resistance in the direction in which the tip connector 4 is to be pushed during said connecting operation.

The external urinary catheter according to the invention may be manufactured in any suitable way.

The tip portion 4 of the catheter may e.g. be manufactured by injection-moulding it into a cavity that is provided with the projections or indentations necessary for producing any of the friction-increasing means of Fig. 1. The other parts of the catheter may be produced in a similar or any other suitable way such as a dipping or extrusion process.

The invention should not be regarded as being limited to the embodiments described in the above but various combinations and modifications may be carried out without departing from the scope of the following claims.

## Claims

1. An external urinary catheter with a longitudinal axis, said catheter being made of silicone and comprising a sheath portion (1) and a tip portion (2) for receiving a connector, said tip portion (2) being attached to one end of said sheath portion (1) and comprising a seizing area (4a) intended for seizing by hand and subsequent guidance of said tip portion (2) into connection with a hose connector, **characterised in that** said seizing area (4a) is disposed on its surface with friction-increasing means (4b) in the form of a plurality circumferential ribs extending about said longitudinal axis.

2. An external urinary catheter according to claim 1, wherein the catheter and friction-increasing means are mould injected.

3. An external urinary catheter according to claim 2, wherein the catheter and friction-increasing means are mould injected in one piece.

4. A method for manufacturing an external urinary catheter according to claim 1 comprising the step of injection-moulding the tip portion (4) of the catheter into a cavity that is provided with the projections or indentations necessary for producing the friction-increasing means.

## Patentansprüche

1. Externer Harnkatheter mit einer Längsachse, wobei der Katheter aus Silicon hergestellt ist und einen Hüllteil (1) und einen Spitzenteil (2) zur Aufnahme eines Verbinders aufweist,
wobei der Spitzenteil (2) an einem Ende des Hüllteils (1) angebracht ist und einen Greifbereich (4a) aufweist, der zum Ergreifen mit der Hand und zum anschließenden Führen des Spitzenteils (2) in eine Verbindung mit einem Schlauchverbinder dient,
**dadurch gekennzeichnet,**
**dass** der Greifbereich (4a) auf seiner Oberfläche mit reibungserhöhenden Mitteln (4b) in Form von mehreren Umfangsrippen versehen ist, die sich um die Längsachse herum erstrecken.

2. Externer Harnkatheter nach Anspruch 1, wobei der Katheter und die reibungserhöhenden Mittel durch Spritzgießen hergestellt sind.

3. Externer Harnkatheter nach Anspruch 2, wobei der Katheter und die reibungserhöhenden Mittel durch Spritzgießen einstückig hergestellt sind.

4. Verfahren zur Herstellung eines externen Harnkatheters gemäß Anspruch 1, das den folgenden Schritt umfasst:
Spritzgießen des Spitzenteils (4) des Katheters in einen Hohlraum, der mit Vorsprüngen oder Vertiefungen versehen ist, die zur Erzeugung der reibungserhöhenden Mittel erforderlich sind.

## Revendications

1. Cathéter urinaire externe avec un axe longitudinal, ledit cathéter étant constitué de silicone et comprenant une partie gaine (1) et une partie terminale (2) permettant de recevoir un raccord, ladite partie terminale (2) étant fixée à une extrémité de ladite partie de gaine (1) et comprenant une surface de prise (4a) permettant à l'utilisateur de saisir et de guider ladite partie terminale (2) reliée à un raccord de tuyau, **caractérisé en ce que** ladite surface de prise (4a) est disposée sur sa surface avec un moyen d'augmenter la zone de frottement (4b) sous la forme d'une pluralité de nervures circonférentielles s'étendant autour dudit axe longitudinal.

2. Cathéter urinaire externe selon la revendication 1, dans lequel le cathéter et le moyen d'augmenter la zone de frottement sont moulés par injection.

3. Cathéter urinaire externe selon la revendication 1, dans lequel le cathéter et le moyen d'augmenter du frottement sont moulés par injection en une seule pièce.

4. Procédé de fabrication d'un cathéter externe selon la revendication 1 comprenant l'étape de moulage par injection de la partie terminale (4) du cathéter dans une cavité qui est munie de saillies ou entailles nécessaires pour réaliser le moyen d'augmenter du frottement.
